# EUROPEAN PATENT APPLICATION

(11) **EP 2 884 282 A1**
(43) Date of publication of application: **17.06.2015**
(21) Application number: 13197278.8
(22) Date of filing: 13.12.2013
(51) Int. Cl.: G01N 33/68

(54) **Use of tryptophan as a biomarker for patient selection, dosing and therapy monitoring for pharmaceutical compositions targeting the intestinal microbiota in diseases featuring tryptophan deficiency**

(71) Applicant: CONARIS Research Institute AG, 24118 Kiel (DE)
(72) Inventor: Wätzig, Georg, 24106 Kiel (DE); Seegert, Dirk, 24229 Dänischenhagen (DE)
(74) Representative: Eisenführ Speiser

(57) **Abstract**

The present invention relates to a novel use of tryptophan as a biomarker for patient selection, dosing and therapy monitoring for pharmaceutical compositions targeting the intestinal microbiota, wherein tryptophan levels are preferably measured in blood, plasma or serum.

## Description

### FIELD OF THE INVENTION

The present invention relates to a novel use of tryptophan as a biomarker for patient selection, dosing and therapy monitoring for pharmaceutical compositions targeting the intestinal microbiota, wherein tryptophan levels are preferably measured in blood, plasma or serum. The present invention also relates to a pharmaceutical composition for treating tryptophan-deficient patients suffering from a disease or condition disclosed herein and the use of nicotinamide and related compounds to treat a disease or condition disclosed herein.

### BACKGROUND

Many inflammatory diseases of the intestinal wall are caused or influenced by changes in the intestinal microbiota and/or an impaired and/or disturbed interaction between the intestinal microbiota and the intestines. Such intestinal inflammations occur in humans, *e.g*., inflammatory bowel disease (IBD), such as Crohn's disease (CD) or ulcerative colitis (UC), but also in other mammals (*e.g*., chronic idiopathic colitis in dogs). These diseases are based on complex immunological processes which are not fully understood. However, changes in, and impaired and/or disturbed interactions of, the intestinal microbiota can also be causative factors in a number of other diseases. Examples include atopic diseases, such as atopic eczema, allergic conditions or asthma (see *e.g*., Bisgaard et al. 2011, J. Allergy Clin. Immunol. 128:646; lebba et al. 2011, Dig. Dis. 29:531; Abrahamsson et al. 2012, J. Allergy Clin. Immunol. 129:434; Candela et al. 2012, BMC Microbiol. 12:95; Olszak et al. 2012, Science 336:489), as well as metabolic diseases with an inflammatory component, such as arteriosclerosis with resulting coronary heart diseases, adiposity or diabetes (Ott et al. 2006, Circulation 113:929; Koren et al. 2011, PNAS 108 Suppl 1:4592; for reviews see Caesar et al. 2010, J. Intern. Med. 268:320; and Vrise et al. 2010, Diabetologia 53:606).

Although the relationship between the intestinal microbiota and various diseases is known, it has not been understood how to influence the microbiota in a way that would beneficially impact associated diseases.

Nicotinic acid (niacin, vitamin B3), nicotinamide (nicotinic acid amide) and/or L-tryptophan have been used for the therapy of niacin deficiency diseases (*e.g*., pellagra) for decades. It is known that pellagra may be accompanied by intestinal inflammation, which is ameliorated after niacin administration, where the therapeutic principle is the elimination of the vitamin deficiency causing the intestinal inflammation (Segal et al. 1986, Int. J. Colorectal Dis. 1:238; and Clayton et al. 1991, Eur. J. Pediatr. 150:498).

It has long been known that reduced levels of tryptophan in blood, plasma or serum - herein referred to as tryptophan levels or systemic tryptophan levels - affect serotonin biosynthesis and can result in depression (Schröcksnadel et al. 2006, Clin. Chim. Acta 364:82; Müller et al. 2011, Neurotox. Res. 19:308). In addition to indicating malnutrition, low tryptophan levels are sometimes observed in the context of chronic immune activation (Schröcksnadel et al. 2006, Clin. Chim. Acta 364:82). There is also anecdotal evidence for reduced tryptophan levels in patients with CD (Beeken 1976, Scand. J. Gastroenterol. 11:735; Gupta et al. 2012, Inflamm. Bowel Dis. 18:1214). However, these two studies in CD had several limitations which did not allow reliable conclusions, including that neither study had the statistical power to establish tryptophan levels as a biomarker for IBD or for the other diseases or conditions contemplated by the present invention. Importantly, Beeken clearly correlated the lower tryptophan levels in his CD case reports with malnutrition (patients ate less, had lost more weight) and not with a fundamental disease-relevant mechanism as revealed by the present invention.

It has been previously reported that topical release of nicotinic acid and/or nicotinamide and/or tryptophan in the lower small intestine and/or colon using different controlled- and delayed-release formulations has a surprising anti-inflammatory effect by influencing the intestinal microbiota (the entirety of all microorganisms in the intestines, in particular the bacteria) (PCT/EP2013/062363).

The mechanism behind this surprising effect has subsequently been shown to involve nicotinamide-induced changes in the secretion pattern of antimicrobial peptides in the intestines, which supports the maintenance and/or regeneration of the normal, healthy intestinal microbiota (Hashimoto et al. 2012, Nature 487:477). Hashimoto *et al.* showed that malabsorption of tryptophan in mice leads to a significantly increased severity of colitis induced by the irritant dextran sodium sulfate (DSS). Dietary supplementation of tryptophan via a transporter not affected by the underlying disease mechanism or dietary supplementation of nicotinamide prevented this increase in colitis. Hashimoto *et al.* demonstrated that the increased susceptibility to severe colitis was due to a changed gut microbiome, which, when transplanted to other mice, also increased colitis severity in the recipients. The detrimental change in the gut microbiome was found to be due to strongly reduced amounts of certain antimicrobial peptides (AMPs), especially alpha-defensins, whose expression in epithelial cells of the terminal ileum were largely controlled by mTOR signalling induced by tryptophan or tryptophan metabolites like nicotinamide. Importantly, the inventors have discovered that the tryptophan transporter protein B⁰AT1 is strongly and significantly down-regulated in patients with IBD, but not in non-specific colitis, suggesting a disease-relevant mechanism and leading to tryptophan starvation of the intestinal mucosa in IBD (see PCT/EP2013/062363 and Example 1 of the present invention).

The anecdotal evidence in the state of the art describes reduced tryptophan levels only as a consequence of malnutrition or upregulation of a tryptophan-catabolising enzyme in the inflamed mucosa of patients with CD (Beeken 1976, Scand. J. Gastroenterol. 11:735; Gupta et al. 2012, Inflamm. Bowel Dis. 18:1214). However, there is an urgent and unmet need for biomarkers for personalised medicine in IBD like CD and in many other diseases or conditions. In contrast to current biomarkers in general and the state-of-the-art use of tryptophan in particular, such personalised medicine biomarkers need to deliver more than just reflecting disease activity. The ideal requirements for such a biomarker are that it enables (1) predictions which patients would benefit particularly from a given prophylactic and/or therapeutic intervention or medication, (2) dose finding for a given medication in individual patients and (3) monitoring of therapy success.

### SUMMARY OF THE INVENTION

The inventors have discovered that the tryptophan transporter protein B⁰AT1 is strongly and significantly down-regulated in patients with IBD, but not in non-specific colitis, suggesting a disease-relevant mechanism and leading to tryptophan starvation of the intestinal mucosa in IBD (see Example 1 of the present invention).

The object of the present invention is therefore to provide a novel biomarker for diseases in humans and animals associated with changes in the intestinal microbiota and/or an impaired and/or disturbed interaction between the intestinal microbiota and intestines and/or for diseases or conditions selected from the group consisting of diseases of the small intestine and/or the large intestine; inflammatory bowel diseases (IBD), Crohn's disease (CD), ulcerative colitis (UC), pouchitis; further chronic diseases of the small and/or large intestine or inflammations of the small and/or large intestine; diversion colitis; infectious enteritis; antibiotic-associated diarrhea such as *C. difficile*-associated diarrhea; infectious colitis; diverticulitis; inflammations which are formed in response to irradiation, antibiotics, chemotherapeutic agents, pharmaceutical products or chemicals; colon carcinoma; lipid metabolism disorders, dyslipidemia, non-alcoholic fatty liver disease (NAFLD), non-alcoholic steatohepatitis (NASH); cardiovascular diseases, arteriosclerosis, atherosclerosis; metabolic syndrome and obesity. These changes in the intestinal microbiota have surprisingly been found to be associated with local intestinal levels of tryptophan and/or its metabolites in the context of inflammation and beyond (see our patent application PCT/EP2013/062363 and Hashimoto et al. 2012, Nature 487:477).

The present invention is based, in part, on the unlikely discovery that systemic tryptophan levels can be accurate predictors of the quality of interactions between the intestinal microbiota and the intestines, and can be indicators of the diseases or conditions described herein. According to the invention, the above problem is therefore solved by methods for using tryptophan as a biomarker and companion diagnostic marker for the use in such diseases or conditions:
(1) to predict the usefulness of therapy and to select subjects with low tryptophan levels for prophylaxis and/or therapy with pharmaceutical compositions which increase the amount of bioavailable nicotinic acid and/or nicotinamide and/or tryptophan in the intestine and, thus, beneficially influence the intestinal microbiota and/or their interaction with the intestines and/or in the diseases or conditions described herein,
(2) to enable and/or facilitate dose finding for such subjects and such pharmaceutical compositions in order to optimise therapeutic dosing,
(3) to monitor therapy success (*e.g*., when to stop or reduce dosing, or when to start dosing again to prevent a relapse of the disease or condition).

However, in the context of the present invention, it is important to differentiate between different types of changes in systemic tryptophan levels. The pharmaceutical compositions described herein and in PCT/EP2013/062363 neither aim for nor need to directly influence systemic levels of nicotinic acid and/or nicotinamide and/or tryptophan for efficacy, as their mechanism of action is primarily topical on the intestinal microbiota and/or the intestinal tissue and/or the interaction between the intestinal microbiota and the intestines. However, systemic levels of tryptophan in diseases or conditions described in the present invention and in PCT/EP2013/062363 may indicate that (1) a subject could benefit particularly from being administered the pharmaceutical compositions of the present invention and of PCT/EP2013/062363 (*e.g*., if the subject has particularly low tryptophan levels), and/or (2) that said compositions are effective in increasing nicotinic acid and/or nicotinamide and/or tryptophan levels in the lumen and/or tissue of the lower small intestine and/or colon.

In an embodiment of the invention, the method comprises (1) measuring a tryptophan level in the body of said subject, such as with an *in vivo* or preferably *ex vivo* assay; (2) measuring a tryptophan level in the body of said subject following an administration of one or more doses of the pharmaceutical compositions described herein to said subject, such as with an *in vivo* or preferably *ex vivo* assay; and (3) comparing the level of tryptophan measured in step (1) with the level of tryptophan measured in step (2); wherein said pharmaceutical composition is determined to be effective if the composition is determined to have beneficially influenced the intestinal microbiota and/or their interaction with the intestines and/or the activity and/or progression of the diseases or conditions described herein; and wherein said pharmaceutical composition is determined not to be effective if the composition is determined not to have beneficially influenced the intestinal microbiota and/or their interaction with the intestines and/or the activity and/or progression of the diseases or conditions described herein.

The present invention also provides a method for determining if a subject has a medical condition that is responsive to a pharmaceutical composition which increases the amount of bioavailable nicotinic acid and/or nicotinamide and/or tryptophan in the intestine and, thus, beneficially influences the intestinal microbiota. This comprises monitoring the effect of said composition by the method set forth above; wherein said condition is determined to be unresponsive to the composition if said composition is not determined to have beneficially influenced the intestinal microbiota and/or their interaction with the intestines and/or the activity and/or progression of the diseases or conditions described herein; or wherein the condition is determined to be responsive to the composition if said composition is determined to have beneficially influenced the intestinal microbiota and/or their interaction with the intestines and/or the activity and/or progression of the diseases or conditions described herein.

Furthermore, the present invention provides a method for evaluating dosage of said pharmaceutical composition, in a subject with a disturbed intestinal microbiota and/or an impaired and/or disturbed interaction between the intestinal microbiota and the intestines. This comprises monitoring the effect of the composition, in the subject, by the method set forth above; wherein the dosage is determined to be sufficient if said composition is determined to have beneficially influenced the intestinal microbiota and/or their interaction with the intestines and/or the activity and/or progression of the diseases or conditions described herein; and wherein the dosage is determined to be insufficient if the composition does not beneficially influence the intestinal microbiota and/or their interaction with the intestines and/or the activity and/or progression of the diseases or conditions described herein.

The present invention further provides a method for preventing or treating a disease or condition that results from detrimental changes in the intestinal microbiota and/or an impaired and/or disturbed interaction between intestinal microbiota and intestines, in a subject, comprising administering a dose of a pharmaceutical composition, to the subject, and monitoring the effect of the pharmaceutical composition by the method set forth above; and, continuing prophylaxis and/or treatment if the pharmaceutical composition beneficially influences the intestinal microbiota and/or their interaction with the intestines and/or the activity and/or progression of the diseases or conditions described herein; or discontinuing prophylaxis and/or treatment or increasing dosage administered if the pharmaceutical composition does not beneficially influence the intestinal microbiota and/or their interaction with the intestines and/or the activity and/or progression of the diseases or conditions described herein.

In an embodiment of the invention, the medical condition is a member selected from the group consisting of the diseases or conditions described herein.

### BRIEF DESCRIPTION OF THE DRAWINGS

Figure 1 shows that tryptophan levels in patients with inflammatory bowel diseases (IBD; all patients with Crohn's disease or ulcerative colitis) were significantly lower (***, p<0.0001) than in healthy control individuals. Data are presented as box plots (median; 1. and 3. quartile, whiskers: 10. and 90. percentile).
Figure 2 shows that tryptophan levels in patients with Crohn's disease (CD) were even significantly lower than in ulcerative colitis (UC) and in healthy control individuals (#, p = 0.044; CD vs. UC). Data are presented as box plots (median; 1. and 3. quartile, whiskers: 10. and 90. percentile).
Figure 3 shows that the reduction in tryptophan levels in patients with Crohn's disease (CD) was correlated with disease activity (**, p = 0.0070). Data are presented as box plots (median; 1. and 3. quartile, whiskers: 10. and 90. percentile).
Figure 4 shows that the reduction in tryptophan levels in patients with ulcerative colitis (UC) was correlated with disease activity (**, p = 0.0077). Data are presented as box plots (median; 1. and 3. quartile, whiskers: 10. and 90. percentile).
Figure 5 shows that female individuals had lower tryptophan levels than male individuals, both in controls (**, p = 0.0097) and patients (**, p = 0.0006). Data are presented as box plots (median; 1. and 3. quartile, whiskers: 10. and 90. percentile).
Figure 6 shows that controlled-release nicotinamide minitablets (NAM; dose: approximately 60 mg/kg) conferred a survival benefit in chronic dextrane sodium sulfate (DSS) colitis in C57BL/6J mice, which had been fed a diet with only 25% of the normal content of tryptophan, nicotinic acid and nicotinamide. Mice were terminated 5 days after the second cycle of DSS treatment.

### DETAILED DESCRIPTION

The present invention provides, for example, the use of tryptophan as a convenient, accurate biomarker and companion diagnostic marker for the use in diseases in humans and animals associated with changes in the intestinal microbiota and/or an impaired and/or disturbed interaction between the intestinal microbiota and intestines and/or the diseases or conditions described herein. The solutions according to the invention are described more in detail below and in the attached claims. In the context of the present invention, tryptophan can be used
(1) to predict the usefulness of therapy and to select subjects with low tryptophan levels for prophylaxis and/or therapy with pharmaceutical compositions which increase the amount of bioavailable nicotinic acid and/or nicotinamide and/or tryptophan in the intestine and, thus, beneficially influence the intestinal microbiota and/or their interaction with the intestines and/or in the diseases or conditions described herein,
(2) to enable and/or facilitate dose finding for such subjects and such pharmaceutical compositions in order to optimise therapeutic dosing,
(3) to monitor therapy success (*e.g*., when to stop or reduce dosing, or when to start dosing again to prevent a relapse of the disease or condition).

For example, declining tryptophan levels in the subject indicate detrimental changes in the intestinal microbiota and/or an impaired and/or disturbed interaction between the intestinal microbiota and intestines; and rising tryptophan levels in the subject indicate beneficial changes in the intestinal microbiota and/or their interaction with the intestines. As a result of successful treatment, the tryptophan levels are supposed rise above the lower value of the range measured in healthy normal subjects, *e.g*.. human subjects. As used herein, the term "rising tryptophan levels" as a beneficial or positive outcome is always defined as "rising up to the normal range measured in healthy normal subjects".

In an embodiment of the invention, subnormal tryptophan levels are below the normal levels relative to the average subject (*e.g*., human). Normal tryptophan levels in the population as well as reduced tryptophan levels in patients are discussed herein. In another embodiment of the invention, subnormal levels are significantly below the healthy, normal levels for the particular subject being evaluated. For example, in certain embodiments, a subject has a tryptophan level that falls in the 25^{th} percentile or less as compared to normal individuals, the 20^{th} percentile or less as compared to normal individuals, the 15^{th} percentile or less as compared to normal individuals, the 10^{th} percentile or less as compared to normal individuals or even the 5^{th} percentile or less as compared to normal individuals. In another example, the absolute concentration of tryptophan in human blood and/or plasma and/or serum is less than 52 µM, 50 µM, 45 µM or even 40 µM. In an embodiment of the invention, subnormal levels are levels that are observed to decrease consistently over time in a patient being evaluated. In such an embodiment, a patient's tryptophan level is measured at an initial time point and measured at one or more points in the future. If one or more of the future measurements is significantly lower than a previous measurement, the patient is determined to exhibit a reduced or subnormal tryptophan level. In an embodiment of the invention, reduced or subnormal levels of tryptophan in a patient are any level that a practitioner of ordinary skill in the art would recognise as such. In an embodiment of the invention, a reduced or subnormal level of tryptophan is at least 10%, preferably at least 15%, 20%, 25%, 30%, 35%, 40%, 45%, 50%, 55%, 60%, wherein each value is more preferred than the preceding value) lower than a normal level.

The fact that tryptophan is a soluble amino acid and the surprising finding of the present invention that systemic tryptophan levels are accurate predictors of the quality of interactions between the intestinal microbiota and the intestines makes the use of tryptophan as a biomarker simple since its levels can be determined in the subject's blood or plasma or serum or other bodily fluids. The need for unduly invasive procedures for obtaining biomarker levels (*e.g*., colonoscopies, or intestinal biopsies obtained by colonoscopies) is eliminated through use of systemic tryptophan levels.

As used herein, "beneficially influencing" the intestinal microbiota and/or their interaction with the intestines refers to causing a change in the intestinal microbiota that has a beneficial impact on health, especially on one or more of the diseases or conditions described herein. For example, beneficial impacts are associated with reducing the number of pathogenic bacteria, reducing the ratio of pathogenic bacteria to beneficial bacteria, increasing the diversity of the microbiota, reducing the amount of inflammation that the microbiota induce in the intestines, and partly or completely reverting pathological changes in the enterotype of the microbiota (*e.g*., enterotypes associated with *Bacteroides, Prevotella* and *Ruminococcus).* In the example of IBD, bacteria generally regarded as pathogenic in IBD include, for example, *Enterobacteriaceae (e.g., Escherichia coli)* with invasive properties or virulence factors, sulphide-producing *Desulfovibrio* spp. and *Fusobacterium* spp with invasive properties. Bacteria generally regarded as beneficial include species from the genera *Lactobacillus, Bifidobacterium* and *Faecalibacterium,* such as *L*. *casei, L. plantarum* and *F. prausnitzii.* For a recent overview of the gut microbiota in IBD, see Manichanh et al. 2012, Nat. Rev. Gastroenterol. Hepatol. 9:599.

The term "subject" refers to a mammal, *e.g*., a human. The claimed substances are equally usable for the therapy or prophylaxis of diseases or conditions with similar genesis in both humans and other mammals, in particular in domestic and useful animals. Examples of such animals are dogs, cats, horses, camels or cows without objective restriction.

The term "tryptophan" refers to the essential amino acid tryptophan.

As used herein, the term "pharmaceutical composition" refers to a pharmaceutical composition comprising an active substance selected from nicotinic acid; nicotinamide; tryptophan; a compound that converts in the body of an animal or human into nicotinic acid, nicotinamide or tryptophan; nicotinamide adenine dinucleotide (NAD); nicotinamide adenine dinucleotide phosphate (NADP); an intermediate in the biosynthesis of NAD or NADP; a tryptophan dipeptide; or a combination thereof for beneficially influencing the intestinal microbiota, wherein the pharmaceutical composition releases the active substance preferably for topical efficacy in the lower small intestine, preferably in the terminal ileum, and/or in the colon. The pharmaceutical composition can be used for the therapy or prophylaxis the diseases or conditions described herein, in particular of diseases of the small intestine and/or the large intestine, IBD, Crohn's disease (CD), ulcerative colitis (UC), pouchitis, colon carcinoma, lipid metabolism disorders, dyslipidemia, non-alcoholic fatty liver disease (NAFLD), non-alcoholic steatohepatitis (NASH), cardiovascular diseases, arteriosclerosis, atherosclerosis, metabolic syndrome, obesity, and other diseases which partly or entirely result from changes in the intestinal microbiota and/or an impaired and/or disturbed interaction between intestinal microbiota and intestines and wherein these diseases are attended by a tryptophan level which is lower than the normal level measured in healthy subjects.

As mentioned above, the diseases described herein are selected from the group consisting of diseases of the small intestine and/or the large intestine; inflammatory bowel diseases, Crohn's disease, ulcerative colitis, pouchitis; further chronic diseases of the small and/or large intestine or inflammations of the small and/or large intestine; diversion colitis; infectious enteritis; antibiotic-associated diarrhea such as *C. difficile*-associated diarrhea; infectious colitis; diverticulitis; inflammations which are formed in response to irradiation, antibiotics, chemotherapeutic agents, pharmaceutical products or chemicals; colon carcinoma; lipid metabolism disorders, dyslipidemia, non-alcoholic fatty liver disease (NAFLD), non-alcoholic steatohepatitis (NASH); cardiovascular diseases, arteriosclerosis, atherosclerosis; metabolic syndrome, obesity; and other diseases or conditions which partly or entirely result from changes in the intestinal microbiota and/or an impaired and/or disturbed interaction between intestinal microbiota and intestines.

As used herein, the mention of one pharmaceutical composition of the group described herein always represents all compositions of this group and is only used for reasons of conciseness and legibility.

As used herein, the terms "treatment", "treat", and "treating" refer to reversing, alleviating, delaying the onset of, or inhibiting the progress of a disease or disorder or condition, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (*e.g*., in light of a history of symptoms and/or in light of genetic or other susceptibility factors). Treatment may also be continued after symptoms have resolved, for example to prevent or delay their recurrence.

As used herein, the terms "prophylaxis" and "prevent" refer to delaying the onset of or reducing the likelihood of developing a disease or disorder or condition or one or more symptoms thereof, as compared to an untreated control population.

As used herein, the term "topical efficacy" refers to a topical effect, in the pharmacodynamic sense, and thus refers to a local, rather than systemic, target for a medication. Accordingly, local efficacy means a local therapy and/or prophylaxis of an active substance specifically or selectively to a location where the medication shall deliver its direct therapeutic and/or prophylactic effect and does not or only to a low degree enter the circulatory system, *e.g*., thereby not causing any or only a low systemic action. In this regard, the topical efficacy of the present invention is also contrasted with enteral (in the digestive tract) and intravascular/intravenous (injected into the circulatory system) administrations. Preferably, in the context of the present invention, topical efficacy means that blood and/or plasma and/or serum levels of the active substance and/or metabolites do not exceed levels which are two orders (preferably one order) of magnitude higher than the levels measured in the same person before dosing,

Topical efficacy is achieved in particular by the pharmaceutical formulations of the active substances as described herein and in PCT/EP2013/06263.

The present invention provides a method for monitoring the effect of a pharmaceutical composition on the intestinal microbiota and/or their interaction with the intestines, in the body of a subject suffering from or at risk for detrimental changes in the intestinal microbiota and/or an impaired and/or disturbed interaction between intestinal microbiota and intestines and/or suffering from a disease or condition described herein, which subject is administered said composition, comprising evaluating tryptophan levels in the body of the subject over time; wherein declining tryptophan levels in the subject indicate detrimental changes in the intestinal microbiota and/or an impaired and/or disturbed interaction between the intestinal microbiota and intestines; and rising tryptophan levels in the subject indicate beneficial changes in the intestinal microbiota and/or their interaction with the intestines.

Accordingly, part of the invention is a method for monitoring the efficacy of prophylaxis or treatment of a disease or condition selected from the group consisting of diseases of the small intestine and/or the large intestine; inflammatory bowel diseases, Crohn's disease, ulcerative colitis, pouchitis; further chronic diseases of the small and/or large intestine or inflammations of the small and/or large intestine; diversion colitis; infectious enteritis; antibiotic-associated diarrhea such as *C. difficile*-associated diarrhea; infectious colitis; diverticulitis; inflammations which are formed in response to irradiation, antibiotics, chemotherapeutic agents, pharmaceutical products or chemicals; colon carcinoma; lipid metabolism disorders, dyslipidemia, non-alcoholic fatty liver disease (NAFLD), non-alcoholic steatohepatitis (NASH); cardiovascular diseases, arteriosclerosis, atherosclerosis; metabolic syndrome, obesity; and other diseases or conditions which partly or entirely result from changes in the intestinal microbiota and/or an impaired and/or disturbed interaction between intestinal microbiota and intestines;
wherein the prophylaxis or treatment comprises
administering a pharmaceutical composition comprising an active substance selected from nicotinic acid; nicotinamide; tryptophan; a compound that converts in the body of an animal or human into nicotinic acid, nicotinamide or tryptophan; nicotinamide adenine dinucleotide (NAD); nicotinamide adenine dinucleotide phosphate (NADP); an intermediate in the biosynthesis of NAD or NADP; a tryptophan dipeptide; or a combination thereof to a subject, comprising the steps of
(a) determining the level of tryptophan in blood and/or plasma and/or serum and/or other bodily fluids, and
(b) making a decision for
(b1) continuing the prophylaxis or treatment if the tryptophan level is less than 90% of the normal level observed in healthy subjects of the same species or
(b2) discontinuing the prophylaxis or treatment if the tryptophan level has reached a level which is more than 90% of the normal level observed in healthy subjects of the same species, and
(c) optionally repeating step (a) after discontinuation of the prophylaxis or treatment preferably in regular intervals to detect whether the tryptophan level has decreased again below 90% of the normal level observed in healthy subjects of the same species, and
(d) optionally recommencing the prophylaxis or treatment if in step (c) such a decrease was determined, and
(e) optionally repeating steps (a), (b), (c) and (d) as appropriate.

According to the invention, it is preferred that a monitoring of the efficacy of the prophylaxis or treatment is continued after the prophylaxis or treatment was stopped. It is preferred to perform such monitoring in regular intervals, *e.g*., every week, once a month, once in three months or once in a year. For example, such monitoring can aid a physician to decide to continue the prophylaxis or therapy in a subject, and the physician can get a hint if the disease or condition described herein that came along with a lowered tryptophan level is at least partly cured, which often may be due to a improved intestinal microbiota.

The mean normal level of tryptophan in the blood and/or plasma and/or serum of a healthy human subject is in the range of 1.2-1.8 mg/dL (approximately 58-88 µM; source: German reference laboratory, Medizinisches Labor Bremen, www.mlhb.de), whereas, *e.g*., some patients with IBD or other inflammatory diseases have been reported to have significantly lower levels (Schröcksnadel et al. 2006, Clin. Chim. Acta 364:82; Gupta et al. 2012, Inflamm. Bowel Dis. 18:1214). The only anecdotal case study the inventors have found in the literature on tryptophan levels and their correlation with disease activity in CD (Gupta et al. 2012, Inflamm. Bowel Dis. 18:1214) reported lower values, namely mean levels of 33.8 µM (0.7 mg/dL) in healthy subjects and down to 12.7 µM (0.3 mg/dL in CD patients with severe disease activity. However, our own data (see Example 2) are in a similar range as the normal reference range cited above.

For the determination if a tryptophan level is lower than that of a healthy subject, in case of doubt, a concentration of 58 µM tryptophan in the respective fluid is used as the reference value for a healthy subject (normal level). It may be preferred to differentiate between man and woman. In this case, the aforementioned level would be 58 µM for men and 52 µM for women.Preferably, the tryptophan levels are measured *in vitro* using commercial HPLC and ELISA kits, preferably HPLC, (*e.g*. for EDTA-treated human plasma) or LC/MS (*e.g*., for urine or liquor).

In an embodiment of the invention, an increase of tryptophan levels in response to prophylaxis and/or treatment with a pharmaceutical composition as described herein to any significant degree (*i.e*., an increase of at least 2 µM, preferably 4 µM and more preferably 6 µM), would indicate that the intestinal microbiota and/or their interaction with the intestines is also being beneficially influenced by the prophylaxis and/or treatment.

In an embodiment of the invention, tryptophan rises from a reduced level to a normal level. A qualitative assessment of a rise in tryptophan, in response to a pharmaceutical composition as described herein, by *e.g*., a treating physician or clinician, can also be done so as to assess whether the beneficial effect of said pharmaceutical composition is sufficient; methods including such embodiments are within the scope of the present invention. In the expert, experienced judgment of the physician or clinician, an assessment as to whether the rise was sufficient and indicative of prophylactic or therapeutic efficacy can be made.

In an embodiment of the present invention, tryptophan levels are determined by standard laboratory techniques, which are known to the person skilled in the art. Tryptophan levels can be, in an embodiment of the invention, determined in, *e.g*., the blood or serum or plasma or any other appropriate bodily fluid of a subject or in intestinal tissue.

The present invention also provides a method for determining changes in the intestinal microbiota and/or their interaction with the intestines in a subject; comprising determining tryptophan levels in said subject (*e.g*., over time, *e.g*., in several measurements of tryptophan); wherein declining tryptophan levels, in the subject, indicate detrimental changes in the microbiota and/or their interaction with the intestines; or that the pharmaceutical composition (that was administered to the subject) is not beneficially influencing the intestinal microbiota and/or their interaction with the intestines; and wherein rising tryptophan levels, in the subject, indicate beneficial changes in the microbiota and/or their interaction with the intestines; or that the pharmaceutical composition (that was administered to the subject) is beneficially influencing the intestinal microbiota and/or their interaction with the intestines.

Such a method can be coupled with administration of a pharmaceutical composition as described herein, and, then, evaluation of the intestinal microbiota and/or their interaction with the intestine and/or the activity and/or progression of the diseases or conditions described herein. Such a method serves to determine the effect of said composition.

In an embodiment of the invention, the method comprises (1) measuring a tryptophan level in the body of said subject, such as with an *in vivo* or preferably *ex vivo* assay; (2) measuring a tryptophan level in the body of said subject following an administration of one or more doses of the pharmaceutical compositions described herein to said subject, such as with an *in vivo* or preferably *ex vivo* assay; and (3) comparing the level of tryptophan measured in step (1) with the level of tryptophan measured in step (2); wherein said pharmaceutical composition is determined to be effective in beneficially influencing the intestinal microbiota and/or their interaction with the intestines and/or the activity and/or progression of the diseases or conditions described herein if tryptophan levels are observed to increase over time following said administration; or wherein said pharmaceutical composition is determined not to be effective in beneficially influencing the intestinal microbiota and/or their interaction with the intestines and/or the activity and/or progression of the diseases or conditions described herein if tryptophan levels are observed not to increase over time following said administration. If said composition is determined to be satisfactory regarding beneficially influencing the intestinal microbiota and/or their interaction with the intestines and/or the activity and/or progression of the diseases or conditions described herein, then, optionally, the clinician can elect to continue treatment with the composition. If said composition is not determined to be satisfactory, the clinician can elect to discontinue treatment or, alternatively, alter the composition's dosage (*e.g*., increase dosage) and, then, re-evaluate the effect of the composition.

The methods for monitoring the effect of said composition can be applied, for example, in a method for determining if a subject has a medical condition (*e.g*., a medical condition partly or entirely resulting from detrimental changes in the intestinal microbiota and/or an impaired and/or disturbed interaction between intestinal microbiota and intestines and/or a disease or condition described herein) that is responsive to a pharmaceutical composition as described herein. For example, in such an embodiment of the invention, the subject's tryptophan levels are assayed during the course of treatment with said composition (as discussed herein); and the condition is determined to be unresponsive to said composition if the tryptophan levels are not observed to rise over time following administration of said composition; or wherein the condition is determined to be responsive to said composition if the tryptophan levels are observed to rise over time following administration of said composition.

For example, in an embodiment of the invention, the method comprises: (1) measuring a tryptophan level in the body of said subject, such as with an *in vivo* or preferably *ex vivo* assay; (2) measuring a tryptophan level in the body of said subject following an administration of one or more doses of the pharmaceutical compositions described herein to said subject, such as with an *in vivo* or preferably *ex vivo* assay; and (3) comparing the level of tryptophan measured in step (1) with the level of tryptophan measured in step (2); wherein said disease or condition is determined to be unresponsive to said agent if the tryptophan levels are not observed to rise over time following said administration; or wherein the disease or condition is determined to be responsive to said composition if the tryptophan levels are observed to rise over time following said administration. Optionally, a treating clinician can elect to initiate or continue treatment if said composition is determined to have beneficially influenced the intestinal microbiota and/or their interaction with the intestines and/or the activity and/or progression of the diseases or conditions described herein. The clinician can opt to discontinue or forego treatment with the composition if the disease and/or condition is non-responsive to the composition.

The methods for monitoring the effect of a pharmaceutical composition as described herein may also be used in a method for evaluating dosage of said composition comprising monitoring the effect of the composition by the method set forth above; wherein the dosage is determined to be sufficient if the tryptophan level is observed to rise over time following administration of a dose of the composition; or, wherein the dosage is determined to be insufficient if the tryptophan level is observed to decline over time or remain constant following a dose of the composition. For example, in an embodiment of the invention, the method comprises: (1) measuring a tryptophan level in the body of said subject, such as with an *in vivo* or preferably *ex vivo* assay; (2) administering one or more doses of the pharmaceutical compositions described herein to said subject; (3) measuring a tryptophan level in the body of said subject following said administration such as with an *in vivo* or preferably *ex vivo* assay; and (4) comparing the level of tryptophan measured in step (1) with the level of tryptophan measured in step (3); wherein the dosage is determined to be sufficient if the tryptophan level is observed to rise over time following administration of a dose of the composition or wherein the dosage is determined to be insufficient if the tryptophan level is observed to decline over time or remain constant following a dose of the composition. Optionally, the treating clinician can opt to increase dosage if the dosage is determined to be insufficient; or, to maintain dosage if the dose evaluated is determined to be effective.

In addition, the method for monitoring the effect of a composition as described herein can be applied in a method for treating a disease or condition as described herein in a subject. Such a method comprises administering a dosage of said composition to the subject and monitoring the effect of the composition by the monitoring method discussed above; and increasing the dosage of the composition if the tryptophan levels are determined to decline over time or remain constant following a dosage of the composition or maintaining dosage if tryptophan levels are determined to rise over time. For example, an embodiment of the invention includes the following steps: (1) measuring a tryptophan level in the body of said subject, such as with an *in vivo* or preferably *ex vivo* assay; (2) measuring a tryptophan level in the body of said subject following an administration of one or more doses of the pharmaceutical compositions described herein to said subject, such as with an *in vivo* or preferably *ex vivo* assay; and (3) comparing the level of tryptophan measured in step (1) with the level of tryptophan measured in step (2); and increasing the dosage of the composition if the tryptophan levels are determined to decline over time or remain constant following a dosage of the composition, or maintaining dosage if tryptophan levels are determined to rise over time.

The effect of a pharmaceutical composition as described herein can be evaluated at the outset of or in the midst of a treatment regimen. Specifically, in connection with any of the methods set forth above, the subject may have been administered one or more doses of said composition before an initial measurement of tryptophan, for example, prior to step (1) in the methods set forth above. In such an embodiment, tryptophan levels are determined during the course of an already initiated treatment regimen. In another embodiment of the invention, the dose is an initial, first dose in a treatment regimen. In this case, the subject has not been administered any previous doses in the regimen.

The present invention provides methods for quickly and conveniently evaluating various aspects of a given therapeutic regimen using a pharmaceutical composition as described herein. In an embodiment of the invention, said composition is administered to a patient at a "therapeutically effective dosage" or "therapeutically effective amount" which preferably beneficially influences the intestinal microbiota and/or their interaction with the intestines and/or the activity and/or progression of the diseases or conditions described herein to any extent. As discussed herein, the dosage can be adjusted according to observations made by the clinician, physician or veterinarian based, at least in part, on the behaviour of tryptophan levels in the body of the subject during a course of treatment with said composition. For example, as a starting point, a dosage of a composition of the present invention for the treatment of IBD can feature, *e.g*., an active substance content of 1 - 3000 mg nicotinamide per finished dosage form for oral application and/or 10 - 5000 mg nicotinamide per finished dosage for rectal application.

Active substances, preferably nicotinic acid and/or nicotinamide and/or tryptophan, may be used in any form available on the market, *e.g*., produced by Merck KgaA. Tryptophan may be used as a single amino acid or dipeptide, *e.g*., as a Gly-Trp dipeptide.

In addition to nicotinic acid, nicotinamide and tryptophan, other related compounds can be used in the invention described herein as active substances. For example, compounds that convert into one of these agents (*e.g*., by hydrolysis, metabolism) in the human or animal body are suitable, such as nicotinic acid esters. In addition, intermediates in the synthesis of nicotinamide adenine dinucleotide (NAD) or NAD phosphate (NADP), such as N-formylkynurenine, L-kynurenine, 3-hydroxy-L-kynurenine, 3-hydroxyanthranilate, 2-amino-3-carboxymuconate semialdehyde, quinolinate, and beta-nicotinate D-ribonucleotide, can be used. Further examples include NAD and NADP.

The active substances above can be used alone or in combination with another active agent, such as, *e.g*., 5-aminosalicylic acid (5-ASA, also known as mesalazine or mesalamine), 5-ASA prodrugs (*e.g*., sulfasalazine, balsalazide) or statins. More about such combinations is described in the co-pending application EP application No. EP13197261.4 entitled "A pharmaceutical composition containing combinations of nicotinamide and 5-aminosalicylic acid for beneficially influencing the intestinal microbiota" and a further co-pending application entitled "A pharmaceutical composition containing nicotinic acid and/or nicotinamide for beneficially influencing blood lipid levels by modifying the intestinal microbiota" (both also filed on 13 December 2013 for the same applicant and having the same inventors as the present application), the contents of which are incorporated herein by reference.

Pharmaceutical compositions which contain nicotinic acid and/or nicotinamide and/or tryptophan (or one of the other substances described above), can be administered orally with a retarded, *e.g*. controlled and/or delayed, active substance release or also via a rectal mode of application (*e.g*., enemas or suppositories). The site of delivery of the active substance is preferably the lower small intestine and/or the colon (more preferably the terminal ileum and/or the colon) for modifying the gut microbiota and their interaction with the intestines, and thus differs fundamentally from modes of applications which - *e.g*., for the therapy of pellagra - pursue maximal absorption and metabolism in the organism and, thus, a systemic effect. In addition, the mode of administration according to the invention and the dosage according to the invention minimise the probability for the occurrence of side effects, for example as described in connection with the systemic administration of nicotinic acid.

In this regard, the present invention also comprises combination preparations of the active substances of the present invention, such as a variable dose combination or a fixed dose combination. The combination described herein may be present in the same or separate dosage forms, which may be administered simultaneously or sequentially.

As used herein, the term "variable dose combination" refers to a drug/drug combination of two or more active substances whereby each of these substances is applied in the form of a separate pharmaceutical composition, *e.g*., two single dosage forms, which separate pharmaceutical composition may be administered together by consecutive or subsequent administration regimen. For example, a pharmaceutical composition of nicotinic acid in any suitable dosage thereof may be administered together, consecutively or subsequently, with a separate pharmaceutical composition of nicotinamide in any suitable dosage thereof. Thus, variable dosages of one active substance, *e.g*., of nicotinic acid, may be combined with variable dosages of another active substance, *e.g*., of nicotinamide. These variable dose combinations may use conventionally available pharmaceutical compositions or may be also achieved by customized polypharmacy via compounding.

In contrast to a variable dose combination, a fixed-dose combination is a combination drug which is a formulation including two or more active pharmaceutical ingredients, *e.g*., active substances, combined in a single dosage form, which is manufactured and distributed in certain respective fixed doses. A fixed-dose combination mostly refers to a mass-produced product having a predetermined combination of drugs (active substances) and respective dosages (as opposed to customized polypharmacy via compounding).

Thus, the invention also pertains to embodiments of a pharmaceutical composition or the use thereof as described herein, wherein the pharmaceutical composition is a controlled and/or delayed release formulation of nicotinic acid and/or nicotinamide and/or tryptophan alone, or a variable dose combination or a fixed dose combination of a controlled and/or delayed release formulation of nicotinic acid with a controlled and/or delayed release formulation of nicotinamide and/or with a controlled and/or delayed release formulation of tryptophan.

As used herein, the "lower small intestine" is the second half of the small intestine and the "terminal ileum" is the second half of the ileum.

In order to produce orally administered formulations of an active substance having an anti-inflammatory and/or modifying effect on the intestinal microbiota in the lower small intestine and/or colon, preferably in the terminal ileum and/or in the colon, it is thus advantageous and innovative to use controlled and/or delayed modes of release. In contrast to conventional (in some cases also delayed) modes of release for optimum supplementation, *e.g*., in the case of pellagra, certain embodiments of the present invention partially or substantially avoid an absorption in the stomach and in the upper portions of the small intestine.

In order to treat the diseases or conditions described herein, oral and/or rectal modes (*e.g*., as enema) of application are suitable. For example, in order to treat pouchitis in the case of ulcerative colitis, the rectal application (*e.g*., as enema) is preferred. It can also be supported by an oral administration of the oral formulations described above, *e.g*., delayed releasing preparations. For the symptomatic therapy of any other form of colitis, both the oral and rectal applications can be chosen for the therapeutic modification of the intestinal microbiota. The oral application is preferred for the prophylaxis of the colon carcinoma, in particular in the case of ulcerative colitis, and for the therapy and/or prophylaxis of other diseases or conditions described herein which partially or substantially result from changes in the intestinal microbiota and/or an impaired or disturbed interaction between intestinal microbiota and the intestines.

For oral administration, particular dosage forms that control and/or delay the release of the active substance due to special galenics (so-called controlled release, slow release or delayed release forms) are particularly suitable. Such dosage forms may be simple tablets and also coated tablets, *e.g*., film tablets or dragees. The tablets are usually round or biconvex. Oblong tablet forms, which allow the tablet to be separated, are also possible. In addition, granules, spheroids, pellets or microcapsules are possible, which are filled in sachets or capsules, where appropriate.

The term "delayed release" relates preferably to a pharmaceutical formulation that releases the active ingredients after a period of delay. In certain embodiments, the delay is sufficient for at least a portion of the active substances in a formulation to release in the lower small intestine (*e.g*., terminal ileum) and/or colon.

The term "controlled release" refers preferably to a pharmaceutical formulation or component thereof that releases, or delivers, one or more active ingredients over a prolonged period of time (time-dependent release) and/or under certain physiological conditions (*e.g*., pH-dependent release). In certain embodiments, the period of time or the release according to physiological conditions (*e.g*., pH) is sufficient for at least a portion of the active substances in a formulation to release in the lower small intestine (*e.g*., terminal ileum) and/or colon.

The retardation and/or delayed release and/or controlled release is advantageously achieved, *e.g*., by coatings which are resistant to gastric juice and dissolve depending on the pH, by means of microcellulose and/or multi matrix (MMX) technologies, by using different carrier matrices or a combination of these techniques. Examples include film coatings which contain acrylic and/or methacrylate polymers in various mixtures for controlled and/or delayed release. For example, the active substance(s) can be contained in a conventional matrix of microcrystalline cellulose or gelatin or with MMX technology, which is coated with a material, which provides the delayed release of the active substance(s). An active substance can be administered in large-volume capsules (*e.g*., gelatin capsules having a content of 0.68 ml), which are coated by means of known methods. Suitable coating agents are water-insoluble waxes, such as carnauba wax, and/or polymers, such as poly(meth)acrylates [*e.g*., the poly(meth)acrylate product portfolio with the trade name Eudragit^{®}, in particular Eudragit^{®} L 30 D-55 (an aqueous dispersion of anionic polymers with methacrylic acid as a functional group), Eudragit^{®} L 100-55 (which contains an anionic copolymer based on methacrylic acid and ethyl acrylate), Eudragit^{®} L 100 or L 12,5 or S 100 or S 12,5 (anionic copolymers based on methacrylic acid and methyl methacrylate), or Eudragit^{®} FS 30 D (an aqueous dispersion of an anionic copolymer based on methyl acrylate, methyl methacrylate and methacrylic acid); Evonik Industries AG, Essen, Germany) and/or water-insoluble celluloses (*e.g*., methyl cellulose, ethyl cellulose). Where appropriate, water soluble polymers (*e.g*., polyvinylpyrrolidone), water-soluble celluloses (*e.g*., hydroxypropylmethyl cellulose or hydroxypropyl cellulose), emulsifiers and stabilisers (*e.g*., polysorbate 80), polyethylene glycol (PEG), lactose or mannitol can also be contained in the coating material.

For example, a combination of Eudragit^{®} S and L compounds (*e.g*., Eudragit^{®} L/S 100) effects a controlled release of the active substances according to the invention at pH > 6.4, which occurs in the terminal ileum. Further uses of Eudragit^{®} preparations and mixtures thereof (FS, L, S and R compounds) are also conceivable for the packaging of an active substance, and therefore a topical use in selected portions of the entire gastrointestinal tract can be achieved by controlled release at certain pH values. A systematic study of enteric targeting with hydroxypropyl methylcellulose (HPMC) capsules and more recently developed Eudragit^{®} polymers was published by Cole *et al.* in 2002 (Int. J. Pharm. 231:83).

The pharmaceutical composition described herein can also contain further pharmaceutical excipient substances, such as binders, fillers, glidants, lubricants and flow regulating agents. The compounds according to the invention can be formulated, where appropriate, together with further active substances and with excipients conventional in pharmaceutical compositions, *e.g*., talcum, gum arabic, lactose, starch, magnesium stearate, cocoa butter, aqueous and non-aqueous carriers, lipid components of animal or vegetable origin, paraffin derivatives, glycols (in particular polyethylene glycol), various plasticizers, dispersants, emulsifiers and/or preservatives.

In order to produce enemas or suppositories for rectal application, preparations of an active substance can be dissolved in a suitable solvent and be further processed into enemas or suppositories according to known pharmaceutical methods.

The active substance content in the finished dosage form is 1 - 3000 mg, preferably 10 - 1000 mg, in the case of oral administration; the enemas and/or suppositories can contain an amount of 10 mg to 5000 mg of the active substance. Depending on the intensity and severity of the inflammatory disease, the dosage forms are administered once or several times daily or in another dosage regimen to be chosen by a physician.

A physician or clinician can, optionally, also adjust the dosage of a composition as described herein, using conventional techniques and clinical indicia in addition to tryptophan levels as discussed herein; such additional techniques and indicia are known to the person skilled in the art.

The present invention provides a method for diagnosing the need of a subject, *e.g*., a human patient, for the local supplementation of nicotinic acid and/or nicotinamide and/or tryptophan and/or tryptophan metabolites and/or substances as described herein in the lower small intestine and/or large intestine in order to beneficially influence the intestinal microbiota and/or their interaction with the intestines and/or the activity and/or progression of the diseases or conditions described herein. While the inventors do not wish to be bound by theory, they believe that even a dose of a therapeutic agent that only acts locally (*i.e*., topically) in the gastrointestinal tract may have an indirect impact on systemic tryptophan levels (*e.g*., by correcting a pathological mechanism that leads to lessened tryptophan uptake). The diagnostic method comprises determining if the patient exhibits reduced levels of tryptophan. If the patient is determined to exhibit reduced levels of tryptophan, then the patient has an increased likelihood to benefit from treatment with the compositions described herein. In an embodiment of the invention, the patient is further examined, with additional tests, for changes in the intestinal microbiota and/or impaired and/or disturbed interactions between the intestinal microbiota and the intestines. In an embodiment of the invention, the diagnosis of tryptophan need in the patient as set forth above is confirmed, *e.g*., using conventional techniques. In an embodiment of the invention, diagnosis of tryptophan need in a patient is followed by treatment with a composition as described herein, not for the systemic supplementation of tryptophan, but for locally increasing levels of nicotinic acid and/or nicotinamide and/or tryptophan in the lower small intestine and/or large intestine in order to beneficially influence the intestinal microbiota and/or their interaction with the intestines and/or the activity and/or progression of the diseases or conditions described herein.

### EXEMPLIFICATION

There are variable possibilities to advantageously develop, and develop further, the teaching of the present invention. For this purpose, reference is made to the examples below which describe the invention in a representative way.

### Example 1:

It turned out surprisingly that in patients suffering from Crohn's disease and ulcerative colitis, the expression of the main tryptophan transporter protein B⁰AT1 in inflamed portions of the mucosa was reduced very strongly and in a statistically significant way (P value < 0.05) (see table 1). Interestingly, this reduction was also borderline significant in non-inflamed inflammatory bowel disease (IBD) samples, suggesting several layers of regulation by predisposition or latent changes in the mucosa of IBD patients as well as disease activity. In addition, control samples from patients suffering from intestinal inflammations of different genesis (so called disease specificity controls) showed no significant deviation from hospitalized normal persons (HN). This argues against a general, non-specific deficiency disease or amino acid deficiency and for the specific effects of nicotinic acid, nicotinamide and/or tryptophan (and related compounds) in the treatment of IBD using the compositions claimed in PCT/EP2013/062363 and in the present invention.

**Table 1: mRNA expression of B⁰AT1 with respect to the base value (from samples of hospitalized normal controls, HN, i.e., patients without intestinal inflammation)**

| | Crohn's disease | | Ulcerative colitis | | All IBD samples | All inflamed IBD samples | All non-inflamed IBD samples | Inflamed samples of other intestinal diseases | Non-inflamed samples of other intestinal diseases |
|---|---|---|---|---|---|---|---|---|---|
| | Inflamed | Not inflamed | inflamed | Not inflamed | | | | | |
| Deviation with respect to HN | -8.77 | -1.55 | -31.02 | -2.15 | -5.44 | -18.43 | -1.92 | -1.52 | -1.01 |
| P value | 0.0002 | 0.2772 | 0.0000 | 0.0207 | 0.0000 | 0.0000 | 0.0485 | 0.1093 | 0.3632 |

The tryptophan transporter deficiency observed in IBD patients observed in this example corresponds to the situation in mice with exacerbated colitis due to tryptophan transporter deficiency (Hashimoto et al. 2012, Nature 487:477).

### Example 2:

We investigated tryptophan levels in a large consecutive series of patients with inflammatory bowel diseases (IBD) and healthy controls. From 258 consecutive patients with IBD from an outpatient setting [78 patients with ulcerative colitis (UC), 167 with Crohn's disease (CD), 13 with indeterminate colitis] and 100 consecutive healthy blood donors, a serum sample was prospectively obtained during a four week sampling campaign to assess tryptophan levels. 116 patients were seen twice. Patients were clinically characterized and tryptophan levels were related in an exploratory analysis to clinical characteristics, age, severity of disease, gender, body mass index (BMI), smoking status, C-reactive protein (CRP) levels and duration of disease. A forward and backward logistic regression was performed.

Practically all tryptophan levels in healthy normal human subjects were in the range of 40-80 µM (median: 57.6 µM).

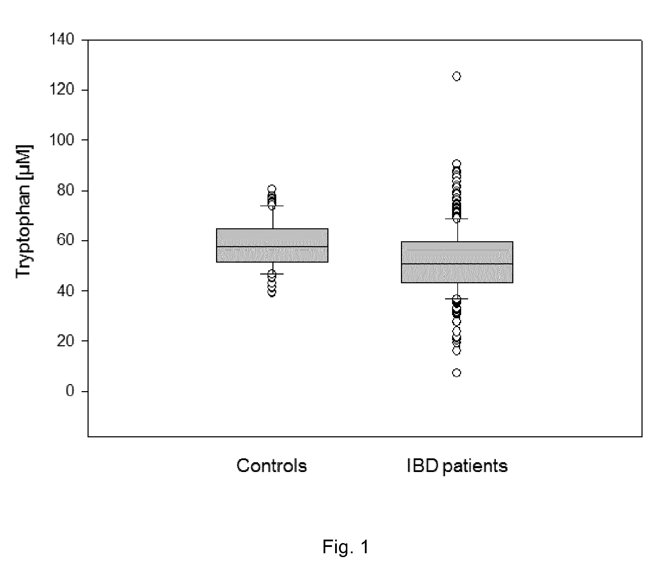

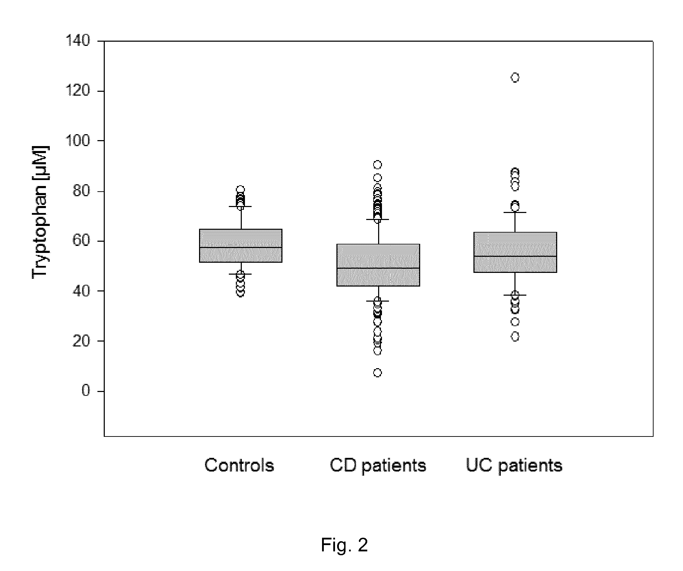

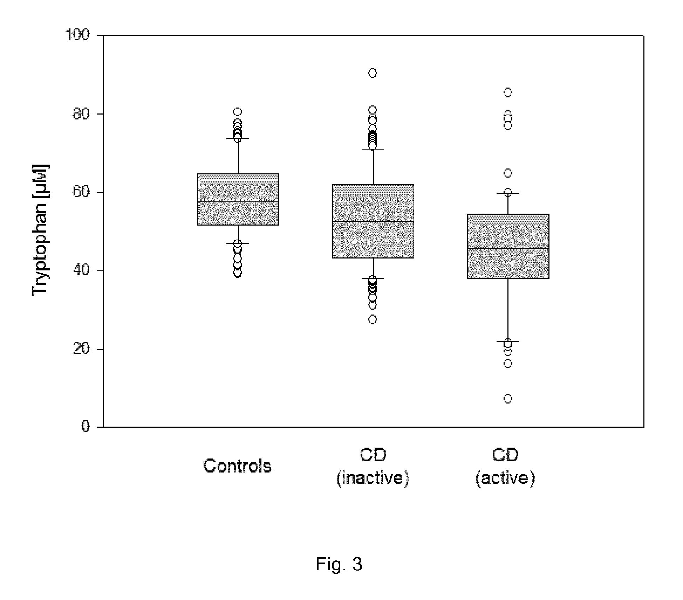

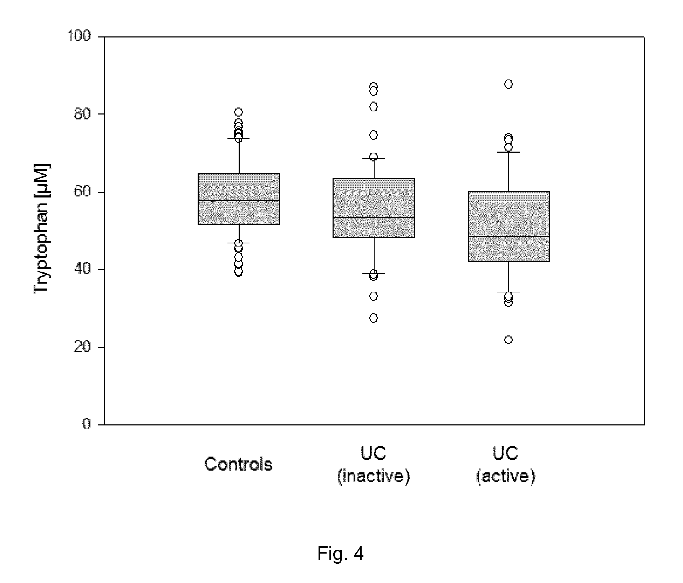

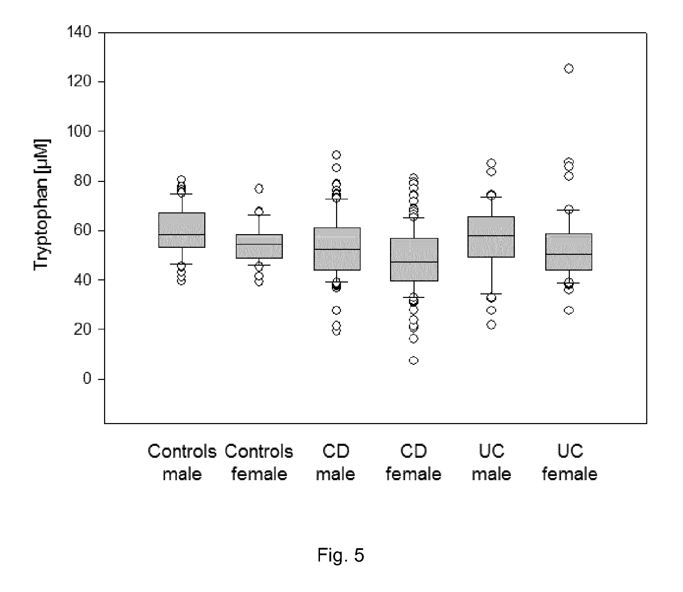

Tryptophan levels in patients with IBD were significantly lower (p<0.0001) than in controls (Figure 1). The signal was stronger in CD patients in whom tryptophan levels were even significantly lower than in UC (p = 0.044) (Figure 2). A clear relationship of low tryptophan levels was seen with high disease activity (p = 0.0070 in CD and p = 0.0077 for UC) (Figures 3 and 4). Female individuals had lower tryptophan levels than male individuals, both in controls (p = 0.0097) and patients (p = 0.0006) (Figure 5). In conclusion, tryptophan deficiency appears to be an important mechanism in IBD.

Taken together with the data from Example 1 and the mechanistic and model data of PCT/EP2013/062363 and Hashimoto et al. 2012 (Nature 487:477), the results of this study provide the last piece of evidence for the following disease-relevant (if not disease-causing) mechanism for IBD, although the inventors do not wish to be bound by this mechanism:
- reduced tryptophan availability in the intestinal mucosa in IBD due to strongly reduced B⁰AT1 transporter expression (which could be a cause and/or a consequence of changed intestinal microbiota and/or the IBD the patient is suffering from; see PCT/EP2013/062363 and Example 1);
- reduced mucosal levels of tryptophan and its metabolites (*e.g*., nicotinamide) lead to signalling changes in the intestinal mucosa (Hashimoto et al. 2012, Nature 487:477);
- these signalling changes lead to a detrimentally changed pattern of secreted antimicrobial peptides (Hashimoto et al. 2012, Nature 487:477);
- the changes in the antimicrobial peptide pattern lead to and/or facilitate and/or enable and/or augment detrimental changes in the intestinal microbiota, resulting in transplantable colitogenic microbiota (Hashimoto et al. 2012, Nature 487:477);
- the colitogenic flora causes and/or exacerbates IBD.

The complete correlation between the disease activity status of the down-regulation of the tryptophan transporter B⁰AT1 expression in the mucosa of patients with IBD and their serum tryptophan levels validates tryptophan as an excellent biomarker for the claimed purposes.

### Example 3:

In order to closely model the situation in human patients with inflammatory bowel diseases (IBD), a murine model for chronic colitis was tested for the first time under conditions of tryptophan reduction (25% of regular tryptophan and nicotinic acid content in the diet), but not complete starvation.

Due to species-specific differences in the gastrointestinal tract in terms of length, passage time and pH milieu, the controlled release formulations were adapted to the organism which was to be treated. Based on the parameters of the murine gastronintestinal tract (Koopman et al. 1978, Lab. Anim. 12:223; McConnell et al. 2008, J. Pharm. Pharmacol. 60:63), a murine-specific formulation was produced for this proof-of-concept study in mice.

Controlled release minitablets were produced with a powder mixed of 99% nicotinamide (NAM) and 1% of magnesium stearate (both from Caelo, Hilden, Germany) as lubricant.

After blending, the powder was characterised in terms of powder flow (angle of repose; <35°) and size distribution (laser diffraction; main particle fraction: 100-200 µm) to ensure good powder flow. Minitablets were then produced in a rotary press and coated by a film of the water-insoluble polymer Kollidon SR 30 D (BASF, Ludwigshafen, Germany) to control NAM release by diffusion of NAM through the film. The coating formulation was as follows: Kollicoat SR 20 D (49.9%), glycerol monostearate 60 (0.743%), propylene glycol (0.743%), red iron oxide (0.4%), polysorbate 80 (0.314%), and water ad 100%.

The glycerol monostearate 60 (Caelo) was heated with half of the water to 80°C and emulsified with an Ultraturrax (IKA, Staufen, Germany). Subsequently, the red iron oxide (Caelo) was added and dispersed for additional 5 min (first bin). The polysorbate 80 (Caelo), the propylene glycol (Caelo) and the polymer dispersions were combined in a second bin and stirred with a magnetic stirrer. The cool (<30°C) emulsion from the first bin was combined with the polymer dispersion from the second bin, and the remaining water was added. The dispersion was stirred for 1 h before filtering (<500 µm). The minitablets were coated in a fluidised bed apparatus (Mycrolab, Hüttlin, Schopfheim, Germany) in a batch size of 50 g with a liquid feed rate of about 1 ml/min and a nebulizing pressure of 0.7 bar. Before spraying, the tablets were pre-heated by a volume flow of 8 m³ at 45°C. During spraying, the volume flow was increased to 16 m³ at 45°C. A product temperature of about 38°C was observed. After spraying, the tablets were fluidised with 16 m³ for additional 10 min at 45°C for curing. In the final process step, the heating was switched off and the tablet bed was cooled to <30°C to avoid sticking. The tablets were coated with 6.2 ± 0.04 mg/cm². Drug release was determined in a paddle apparatus (DT6, Erweka, Heusenstamm, Germany) in according to the Ph. Eur. at 50 rpm. Phosphate buffer (pH 4) was used as dissolution medium because a slightly acid gastrointestinal fluid of about this pH is expected in mice (McConnell et al. 2008, J. Pharm. Pharmacol. 60: 63-70). The drug concentration was determined by UV absorption at 262 nm. The uncoated tablets showed an instantaneous drug release due to the minuscule size of the tablets and the high water solubility of nicotinamide. Using the Kollidon SR coating, the drug release was optimised to cover the target areas in the small intestine of the mice (at least 15 min lag time, constant drug release over 3 h).

The diet of pre-acclimatised male C57BL/6J mice (age 14 weeks) was switched to a custom-made diet with only 25% of the regular content of tryptophan or nicotinic acid or nicotinamide (termed Trp/Nia/NAM-low diet herein), which was produced by mixing a Trp/Nia/NAM-free diet (no tryptophan and 1% of a vitamin premixture without nicotinic acid) in a ratio of 3:1 with a normal diet containing 0.28% tryptophan and 1% of a vitamin premixture with nicotinic acid. Both custom diets were manufactured by Ssniff (Soest, Germany) and were supplied as a powder, which was used to prepare food pellets with either no minitablets (control) or NAM minitablets. The minitablets were homogeneously mixed with the Trp/Nia/NAM-low diet powder, pellets of approximately 2 cm length and 1 cm diameter were formed with a minimum amount of sterile water, frozen in single-use aliquots at -20°C for storage and freshly thawed daily for feeding the mice. The mice received the changed Trp/Nia/NAM-low diet for 2 weeks before the first step of the colitis induction.

The treatment regimen was carried out with two groups of 5 mice each, which were treated as follows:
- Group 1: Trp/Nia/NAM-low diet without minitablets (control).
- Group 2: Controlled release NAM minitablets homogeneously dispersed in the diet (final dose: ca. 60 mg/kg bodyweight, based on a food intake of 2.5 g per mouse per day).

For chronic colitis induction, the mice were supplied with 2.5 % (first cycle) and 3% (second cycle) of dextran sodium sulfate (DSS; molecular mass 40 kDa; TdB consultancy, Uppsala, Sweden) dissolved in the drinking water for 5 days followed by 5 days of regular drinking water. Surviving mice were sacrificed after the 5-day water period of the second DSS cycle (day 21). Survival was monitored daily.

Immediately after termination of the mice, the entire colon was excised, washed with 0.9% saline and "Swiss roll" samples were prepared (Moolenbeek and Ruitenberg 1981: Lab. Anim. 15: 57). In brief, the cleaned colon was cut open longitudinally, wrapped up with the villi facing outward around a small plastic catheter, and the resulting roll was fixed in 4% paraformaldehyde and embedded in paraffin according to standard procedures. The "Swiss roll" preparation enables longitudinal and quantitative histological evaluation of the complete colonic mucosa on the same section slide. Sections were stained with hematoxylin-eosin according to standard procedures. Histological scoring was performed in a blinded fashion. Only optimally conserved and prepared colon samples and sections were used. The histologic score displays the combined score of inflammatory cell infiltration and tissue damage as described elsewhere (Siegmund et al. 2001, Proc. Natl. Acad. Sci. USA 98:13249).

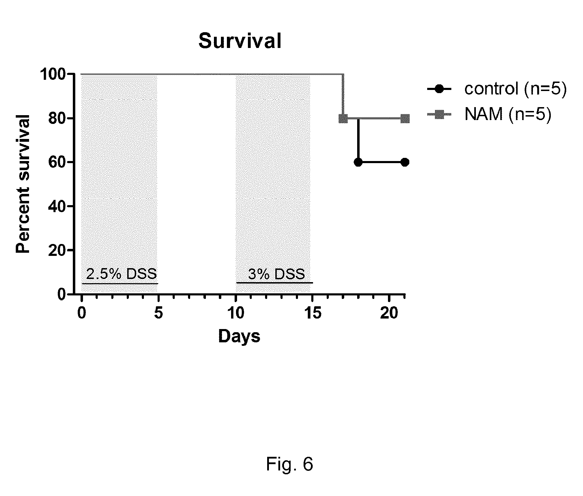

The data of this pilot study show that controlled-release NAM conferred a survival benefit (Figure 6), which is of particular importance as the mice in this experiment were not completely starved of tryptophan, but rather featured a situation resembling the reduced availability of tryptophan and its metabolites in human patients, *e.g*., with IBD (see Example 2). Accordingly, the histological score of the NAM-treated mice was clearly lower (5.0) than the score of the mice in the control group (6.25).

The examples above serve to explain the invention, but are not intended to limit the scope.

## Claims

1. A method for diagnosing a predisposition for or the efficiency of a treatment of a disease or condition selected from the group consisting of diseases of the small intestine and/or the large intestine; inflammatory bowel diseases, Crohn's disease, ulcerative colitis, pouchitis; further chronic diseases of the small and/or large intestine or inflammations of the small and/or large intestine; diversion colitis; infectious enteritis; antibiotic-associated diarrhea such as *C. difficile-*associated diarrhea; infectious colitis; diverticulitis; inflammations which are formed in response to irradiation, antibiotics, chemotherapeutic agents, pharmaceutical products or chemicals; colon carcinoma; lipid metabolism disorders, dyslipidemia, non-alcoholic fatty liver disease (NAFLD), non-alcoholic steatohepatitis (NASH); cardiovascular diseases, arteriosclerosis, atherosclerosis; metabolic syndrome, obesity; and other diseases or conditions which partly or entirely result from changes in the intestinal microbiota and/or an impaired and/or disturbed interaction between intestinal microbiota and intestines; in a subject, comprising determining in a sample of the subject comprising blood and/or plasma and/or serum and/or any other bodily fluid whether the tryptophan level is at least 10% lower than the normal level observed in healthy subjects of the same species, wherein such a lowered tryptophan level is indicative of a predisposition to or the efficacy of the treatment of one of said diseases.

2. The method of claim 1, wherein the treatment comprises administering a therapeutically effective dose of a pharmaceutical composition comprising an active substance selected from nicotinic acid; nicotinamide; tryptophan; a compound that converts in the body of an animal or human into nicotinic acid, nicotinamide or tryptophan; nicotinamide adenine dinucleotide (NAD); nicotinamide adenine dinucleotide phosphate (NADP); an intermediate in the biosynthesis of NAD or NADP; a tryptophan dipeptide; or a combination thereof to the subject.

3. The method of claim 2, wherein the pharmaceutical composition comprising an active substance selected from nicotinic acid; nicotinamide; tryptophan; a compound that converts in the body of an animal or human into nicotinic acid, nicotinamide or tryptophan; nicotinamide adenine dinucleotide (NAD); nicotinamide adenine dinucleotide phosphate (NADP); an intermediate in the biosynthesis of NAD or NADP; a tryptophan dipeptide; or a combination thereof is administered until the tryptophan level reaches the normal level observed in healthy subjects of the same species.

4. A method for monitoring the efficacy of prophylaxis or treatment of a disease or condition selected from the group consisting of diseases of the small intestine and/or the large intestine; inflammatory bowel diseases, Crohn's disease, ulcerative colitis, pouchitis; further chronic diseases of the small and/or large intestine or inflammations of the small and/or large intestine; diversion colitis; infectious enteritis; antibiotic-associated diarrhea such as *C. difficile*-associated diarrhea; infectious colitis; diverticulitis; inflammations which are formed in response to irradiation, antibiotics, chemotherapeutic agents, pharmaceutical products or chemicals; colon carcinoma; lipid metabolism disorders, dyslipidemia, non-alcoholic fatty liver disease (NAFLD), non-alcoholic steatohepatitis (NASH); cardiovascular diseases, arteriosclerosis, atherosclerosis; metabolic syndrome, obesity; and other diseases or conditions which partly or entirely result from changes in the intestinal microbiota and/or an impaired and/or disturbed interaction between intestinal microbiota and intestines;
wherein the prophylaxis or treatment comprises
administering a pharmaceutical composition comprising an active substance selected from nicotinic acid; nicotinamide; tryptophan; a compound that converts in the body of an animal or human into nicotinic acid, nicotinamide or tryptophan; nicotinamide adenine dinucleotide (NAD); nicotinamide adenine dinucleotide phosphate (NADP); an intermediate in the biosynthesis of NAD or NADP; a tryptophan dipeptide; or a combination thereof to a subject,
comprising the steps of
(a) determining the level of tryptophan in blood and/or plasma and/or serum and/or other bodily fluids, and
(b) making a decision for
(b1) continuing the prophylaxis or treatment if the tryptophan level is less than 90% of the normal level observed in healthy subjects of the same species or
(b2) discontinuing the prophylaxis or treatment if the tryptophan level has reached a level which is more than 90% of the normal level observed in healthy subjects of the same species, and
(c) optionally repeating step (a) after discontinuation of the prophylaxis or treatment preferably in regular intervals to detect whether the tryptophan level has decreased again below 90% of the normal level observed in healthy subjects of the same species,
(d) optionally recommencing the prophylaxis or treatment if in step (c) such a decrease was determined, and
(e) optionally repeating steps (a), (b), (c) and (d) as appropriate.

5. The method of any of claims 1 to 4, wherein the determination of the level of tryptophan is performed *in vitro.*

6. A pharmaceutical composition comprising nicotinic acid; nicotinamide; tryptophan; a compound that converts in the body of an animal or human into nicotinic acid, nicotinamide or tryptophan; nicotinamide adenine dinucleotide (NAD); nicotinamide adenine dinucleotide phosphate (NADP); an intermediate in the biosynthesis of NAD or NADP; a tryptophan dipeptide; or a combination thereof for prophylaxis or treatment of a disease or condition selected from the group consisting of diseases of the small intestine and/or the large intestine; inflammatory bowel diseases, Crohn's disease, ulcerative colitis, pouchitis; further chronic diseases of the small and/or large intestine or inflammations of the small and/or large intestine; diversion colitis; infectious enteritis; antibiotic-associated diarrhea such as *C. difficile*-associated diarrhea; infectious colitis; diverticulitis; inflammations which are formed in response to irradiation, antibiotics, chemotherapeutic agents, pharmaceutical products or chemicals; colon carcinoma; lipid metabolism disorders, dyslipidemia, non-alcoholic fatty liver disease (NAFLD), non-alcoholic steatohepatitis (NASH); cardiovascular diseases, arteriosclerosis, atherosclerosis; metabolic syndrome, obesity; and other diseases or conditions which partly or entirely result from changes in the intestinal microbiota and/or an impaired and/or disturbed interaction between intestinal microbiota and intestines; in a subject simultaneously suffering from a tryptophan deficiency and/or reduced and/or subnormal levels of tryptophan in the intestines and in blood and/or plasma and/or serum and/or other bodily fluids.

7. The pharmaceutical composition of claim 6, wherein the subject is human.

8. The pharmaceutical composition of claims 6 or 7, wherein the reduced or subnormal tryptophan level is below the normal level observed in healthy subjects of the same species.

9. The pharmaceutical composition of any one of claims 6 to 8, wherein the dose of one or more active substances selected from nicotinic acid; nicotinamide; tryptophan; a compound that converts in the body of an animal or human into nicotinic acid, nicotinamide or tryptophan; nicotinamide adenine dinucleotide (NAD); nicotinamide adenine dinucleotide phosphate (NADP); an intermediate in the biosynthesis of NAD or NADP; a tryptophan dipeptide; or a combination thereof is 1-3000 mg/day.

10. The pharmaceutical composition of any one of claims 6 to 9, wherein the disease is an inflammatory bowel disease, Crohn's disease, ulcerative colitis or pouchitis.

11. The pharmaceutical composition of any one of claims 6 to 10, wherein the disease is a lipid metabolism disorder, dyslipidemia, non-alcoholic fatty liver disease (NAFLD), non-alcoholic steatohepatitis (NASH), cardiovascular diseases, arteriosclerosis, atherosclerosis, metabolic syndrome or obesity.

12. The pharmaceutical composition of any one of claims 6 to 11, wherein the disease is not an inflammatory bowel disease.

13. The pharmaceutical composition of any one of claims 6 to 12, wherein the tryptophan level in the subject is ≤ 90% of the normal level observed in healthy subjects of the same species.

14. The pharmaceutical composition of claim 13, wherein the tryptophan level in the subject is ≤ 80% of the normal level observed in healthy subjects of the same species.

15. The pharmaceutical composition of any one of claims 6 to 14, wherein the composition is formulated for controlled and/or delayed release and/or topical efficacy in the lower small intestine, preferably in the terminal ileum, and/or in the colon or the method of any one of claims 1 to 5, wherein the pharmaceutical composition to be used for prophylaxis or treatment is formulated for controlled and/or delayed release and/or topical efficacy in the lower small intestine, preferably in the terminal ileum, and/or in the colon.
